(19) Europäisches Patentamt / European Patent Office / Office européen des brevets

(11) **EP 3 389 476 B1**

(12) **EUROPEAN PATENT SPECIFICATION**

(45) Date of publication and mention of the grant of the patent:
**11.09.2019 Bulletin 2019/37**

(21) Application number: **16845371.0**

(22) Date of filing: **16.12.2016**

(51) Int Cl.:
*A61B 5/00* (2006.01)  *A61B 5/01* (2006.01)
*A61B 5/11* (2006.01)

(86) International application number:
**PCT/EP2016/081576**

(87) International publication number:
**WO 2017/103203 (22.06.2017 Gazette 2017/25)**

(54) **METHOD FOR DETERMINING AUTOMATICALLY THE DICHOTOMY INDEX I<O OF AN INDIVIDUAL**

VERFAHREN ZUR AUTOMATISCHEN BESTIMMUNG DES DICHOTOMIE-INDEX I<O EINES INDIVIDUUMS

PROCÉDÉ PERMETTANT DE DÉTERMINER AUTOMATIQUEMENT L'INDICE DE DICHOTOMIE I<O D'UN INDIVIDU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2015 FR 1562557**

(43) Date of publication of application:
**24.10.2018 Bulletin 2018/43**

(73) Proprietors:
- **Université de Technologie de Troyes
  10010 Troyes Cédex (FR)**
- **INSERM - Institut National de la Santé et de la Recherche Médicale
  75654 Paris Cedex (FR)**
- **Altran Technologies - Altran
  92200 Neuilly sur Seine (FR)**
- **Universite Paris-Sud
  91400 Orsay (FR)**

(72) Inventors:
- **CHKEIR, Aly
  10000 Troyes (FR)**
- **DUCHENE, Jacques
  10440 La Riviere De Corps (FR)**
- **HEWSON, David James
  Hertfordshire, Wheathampstead AL4 8AT (GB)**
- **LEVI, Francis, Albert
  94800 Villejuif (FR)**
- **BEAU, Jacques, Jean
  91100 Villabé (FR)**

(74) Representative: **Debay, Damien et al
Debay IP
126 Résidence Elysée 2
18, avenue de la Jonchère
78170 La Celle Saint Cloud (FR)**

(56) References cited:
WO-A1-2014/210588    WO-A1-2015/082382
US-A1- 2008 117 060    US-A1- 2014 215 246

**Description**

**TECHNICAL FIELD OF THE INVENTION**

The invention relates to the field of methods for determining the dichotomy index of an individual, i.e. the index identifying the regularity of the alternation of day activity and night rest as well as their amplitude over 24H giving the possibility of providing a measurement of the circadian rhythm.

**STATE OF THE PRIOR ART**

. Disruptions of the circadian system which may be caused by working in shifts significantly increase the risk of cancer, notably breast, colon and prostate cancer. Also, perturbation of the activity-rest circadian rhythms, measured by actimetry, represents a negative prognostic factor in terms of survival in patients affected with metastatic colon, breast, kidney, ovary or lung cancer, independently of known prognostic factors.

Actimetry consists in a non-invasive technique for measuring the activity/rest cycle and of the sleep-wake rhythm: the circadian rhythm. To do this, an actimeter is used, appearing as a casing provided with at least one accelerometer and which records the successive zero-crossings of the acceleration.

This type of device coupled with a server allowing analysis of the collected data gives the possibility of reliably estimating periods of activity and of sleep, well correlated with those detected by polysomnography, with however greater uncertainty for falling asleep than for awaking.

Although the actimeter is considered as the "gold standard" for measuring activity/rest, it is not ideal for individuals admitted to hospital with a severe pathology and/or which have very reduced mobility, and therefore low physical activity. This is notably due to the fact that the activity levels are considerably lower than those of healthy individuals which makes it extremely difficult to make a distinction between the rest phase and the wakefulness phase, in particular for falling asleep.

The usual methods for detecting activity in this type of individual, adapt the detection thresholds by using parametric or non-parametric algorithms based on smoothing, logical combinations or approaches based on artificial neurone networks as disclosed in Tilmanne, J. "algorithms for sleep-wake identification using actigraphy: a comparative study and new results" Journal of Sleep Research 2009, 18, (1) pp85-98.

In particular, these are neural networks which give the best result. However, in spite of these algorithmic progress, a percentage of errors persists at the detection of the breach between rest and awareness for hospitalized individuals. Other relevant prior art is described in WO 2014/210588 A1, WO 2015/082382 A1, US 2008/0117060 A1, US 2014/0215246 A1.

**DISCUSSION OF THE INVENTION**

The present invention, which is defined in independent claim 1, therefore has the purpose of proposing a method for automatically determining the dichotomy index I<O of an individual, giving the possibility of at least overcoming a portion of the drawbacks of the prior art, by proposing a means for automatically and specifically detecting the activity and rest states on the basis of the dichotomy index which may be applied to all individuals, including persons having very low activity such as elderly persons, or hospitalized persons without using the thresholding methods applied by the prior algorithms.

For this purpose, the invention relates to a method for automatically determining the dichotomy index I<O of an individual, from at least data from a system comprising:

- a portable module composed of at least:

    - a temperature sensor generating a signal of data representative of the body temperature of the individual,
    - an accelerometer simultaneously generating two data signals, a first "ZCM" signal representative of the activity of the individual over time and a second "positionX" signal representative of the tilt of the individual with respect to the ascending vertical over time,

- a computer server provided with at least one processor, one memory, and a display means and configured for receiving the data signals from the portable sensor, and for carrying out said method,

characterized in that the method comprises at least the following steps:

    A. receiving and recording in the memory, by the processor, of the various data of the temperature, ZCM and positionX signals of the portable module,

B. suppressing from the memory by the processor of the non-utilizable data from the module when the latter is not worn by the individual,

C. identifying and resetting to zero by the processor of the aberrant data from the ZCM and positionX signals of the accelerometer and of their recordings in the memory,

D. transforming the values of the positionX signal of the accelerometer by the processor into binary values (0 or 1) corresponding to an alert state (0) or lying-down state (1) of the individual and their recordings in the memory,

E. transforming the values of the ZCM signal of the accelerometer by the processor into binary values (0 or 1) corresponding to an alert or lying-down state of the individual and their recordings in the memory,

F. comparing the binary values of the ZCM and positionX signals of the accelerometer by the processor and identifying at least one lying-down state of the individual constituted by an uninterrupted succession of a binary value identical for the signals of the accelerometer over a time period greater than or equal to 180 minutes,

G. calculating the dichotomy index and its display on the display means, by the processor by following the following equation:

$$I < O = (1 - \frac{NB_C}{NB_L}) \times 100$$

with:

- $NB_C$, the number of values of the first ZCM signal representative of the activity of the individual versus time, located in the lying-down state identified during step E, which are greater than the median of the values of the first ZCM signal representative of the activity of the individual versus time located outside the lying-down state identified during step E.
- $NB_L$, the number of values of the first signal relative to the activity (ZCM) of the individual, located outside the lying-down state identified during step E.

The dichotomy index I<O, represents the percentage of activity, per minute during the rest period (usually at night), which is less than the median of activity away from the bed (usually during daytime). It is a reliable indicator of the activity-rest circadian rhythm. It is calculated from the measurement of the number of accelerations per minute and from the position of the patient by means of a thorax actimeter worn for at least three consecutive days. This is a non-invasive measurement, generally well accepted by the patients.

"ZCM" or the Zero Crossing Mode corresponds to the number of times that the signal passes through 0 for each time period.

"positionX" corresponds to the value of the angle formed by the accelerometer relatively to the ascending vertical when the latter is worn on the chest of the individual.

According to a particularity, the data signals comprise values taken at a regular interval over a time period of 24 hours.

According to another particularity, when the number of values of the data signals for the temperature sensor and the accelerometer are different, the processor carries out a normalization step on the data recorded in the memory prior to step B, by using a cubic spline polynomial interpolation so that each value from the temperature signal is assigned to a value of the positionX and ZCM signals, and said values are recorded in the memory of the system.

According to another particularity, during step B, the processor suppresses from the memory all the values of temperature data strictly less than 30°C and the values of the positionX and ZCM signals which are associated in time with the temperature value strictly less than 30°C.

According to another particularity, during step C, the processor independently applies on the whole of the positionX and ZCM data the following sub-steps:

a) Selecting with the processor in the memory a first block of consecutive values,
b) Sorting in an increasing order the values of the block,
c) Suppressing value repetitions of the block, in order to obtain a unique data set,
d) Calculating the median of the unique data set,
e) Resetting to zero in the memory the values of the positionX and ZCM data signals when their value is greater than the median,
f) Repeating steps a) to e) on the next block of consecutive values.

According to another particularity, the blocks of values are constituted by 31 values corresponding to a time period of 31 minutes of the signals, the median is calculated over the first 10 data of the unique data set and the reset to zero is accomplished by sub-sets of 5 data corresponding to 5 consecutive minutes, if there are less than 2 values greater than the median, then the processor resets to zero in the memory the corresponding sub-set.

According to another particularity, during step D, on the whole of the positionX signal, the processor

- determines and records in the memory, the minimum median by carrying out the following sub-steps:
- sorts the values in an increasing order,
- suppresses the value repetitions, in order to obtain a unique data set, and
- calculates the minimum median over the first 31 values of the unique data set,
- determines and records in the memory, the maximum median by carrying out the following sub-steps:

  - sorts the values in decreasing order,
  - suppresses the value repetitions, in order to obtain a unique data set, and
  - calculates the maximum median over the first 31 values of the unique data set,
  - determines and records in the memory, the threshold median corresponding to the average of the minimum and maximum medians,
  - transforms and records, in the memory, the signal of positionX data by replacing with 0 the values strictly less than the threshold median, and with 1 the values greater than or equal to the latter in order to obtain a positionX_binary data signal,
  - smoothes out the values recorded in the memory of the positionX_binary data signal, by the processor when a set of data delimited by non-identical values corresponds to a time period of less than 1h30, the values of said data set are then reversed, from 0 to 1 or from 1 to 0.

According to another particularity, during step E, on the whole of the ZCM signal, the processor:

- normalizes the ZCM values by dividing each ZCM value by the maximum value of the signal in order to obtain a new ZCM_time signal, the values of which vary between 0 and 1.
- applies to the ZCM_time signal a variance filter over 10 points and records the signal in the memory
- calculates the accumulated quadratic average from the ZCM_time signal,
- searches for a plateau on the data of the accumulated quadratic average by carrying out with the processor the following sub-steps:

  - multiplication of each value of the quadratic average by a factor of $10^4$ and calculation of the rounded to the nearest integer
  - reading with a pitch of 2 units a window corresponding to 200 units,
  - determining the number of points in each window, if the number of found points corresponds to a time period greater than 180 min, the processor increments a "plateau" counter and retains in memory the time index of the first and of the last point of the plateau
  - transforming and recording in the memory, the ZCM data signal by replacing the values located between the time indexes of the plateau with 1 and the other ones with 0 in order to obtain a ZCM_binary data signal.

According to another particularity, during step F, the processor carries out a comparison of the ZCM_binary and positionX_binary data signals in order to determine the time indexes of the plateau for which the initial and final data values correspond to 1, the lying-down state.

According to another particularity, during step G, the processor does not take into account for calculating the dichotomy index, the data corresponding to a time period of one hour before and of one hour after the beginning of the plateau and one hour before and one hour after the end of the plateau.

**SHORT DESCRIPTION OF THE FIGURES**

Other features, details and advantages of the invention will become apparent upon reading the description which follows with reference to the appended figures:

- Fig. 1, illustrates an example of values of the positionX signal (step A),
- Fig. 2, illustrates the same example of values of the positionX signal after suppressing the non-utilizable data due to the fact that the sensor is not worn (step B),
- Fig. 3, illustrates the same example of values of the positionX signal after applying the filter for the aberrant data (step C),
- Fig. 4, illustrates an example of a positionX_binary value corresponding to the same example of values of the positionX signal before smoothing out (step D),

- Fig. 5, illustrates the same example of positionX_binary values corresponding to the same example of values of the positionX signal after smoothing (step D),
- Fig. 6, illustrates an example of a ZCM_time value of the ZCM signal before applying the variance filter (step E),
- Fig. 7, illustrates the same example of a ZCM_time value of the ZCM signal after applying the variance filter (step E),
- Fig. 8, illustrates the same example of a ZCM_time value of the ZCM signal after applying the variance filter with the accumulated quadratic average (step E),
- Fig. 9 illustrates, the ZCM_binary values after transformation of the ZCM signal (step E),
- Fig. 10 illustrates the values required for calculating the dichotomy index
- Fig. 11 illustrates the readout window allowing determination of the existence of a plateau, during step E.

## DETAILED DESCRIPTION OF DIFFERENT EMBODIMENTS OF THE INVENTION

A non-limiting example of an embodiment of the method for automatically determining the dichotomy index I<O of an individual according to the invention is now described. It uses data from a portable module composed of at least:

- a temperature sensor generating a data signal representative of the body temperature of the individual,
- an accelerometer simultaneously generating two data signals, a first ZCM signal, representative of the activity of the individual over time and a second positionX signal representative of the tilt of the individual with respect to the ascending vertical over time.

As an example, the accelerometer used may be the "ADXL345" accelerometer capable of generating a readout of the activity (or "ZCM" Zero Crossing Mode ZCM (zero crossing mode) which corresponds to the number of times during which the signal crosses 0 for each time period) and of the tilt (or "positionX") every minute. The temperature sensor is preferably a sensor of the infrared type configured for measuring the body temperature every 5 minutes.

The portable module may be connected through a wireless communication circuit with a collector module for example through a Bluetooth, WIFI, or GPRS connection. Said collector module communicates the signals to the server. This communication may be GPRS, Bluetooth, WIFI, LIFI, infrared, radio or wired by means of a communication circuit of a computing resources, for example a server including a processing unit, for example a processor, a memory and a program using the data temporarily stored in the memory of the module and issued to the memory of the server through the communication circuit.

In some embodiment, the portable module does not require to be connected to the server. Indeed, the portable module can include the computing resources as a processing unit, for example a processor, a memory and a program using the data from the sensor and accelerometer, temporarily stored in the memory of the module, to perform the method for automatically determining the dichotomy index I<O of an individual. In other words, the portable module and the function of the server can be reunited in one portable element.

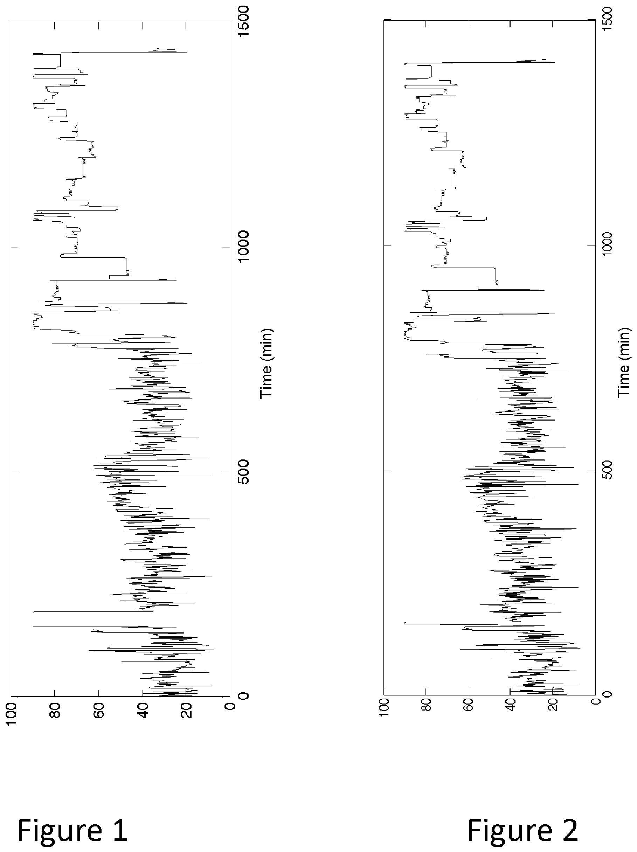

Thus, every 24 hours, the server receives the measurements corresponding to 3 data signals, a first signal corresponding to a succession of representative values of the body temperature of the individual for example taken every 5 minutes over a 24-hour period, (a higher frequency may of course be used but is not necessarily relevant given that the variation of the body temperature is slow), a second "ZCM" signal corresponding to a succession of values representative of the activity of the individual for example taken every minute over a period of 24 hours and a third "positionX" signal (Fig. 1), corresponding to a succession of values representative of the tilt of the individual with respect to the ascending vertical, these values being taken for example every minute over a period of 24 hours.

A "24-hours period" is understood to mean: a period of 24 hours which can include a corrective factor (which can be added to, or subtracted from, this time period) of the order of a few minutes to a maximum of the order of one hour. This correction factor may for example be calculated on the basis of previous measurements for an individual, in order to determine the duration of these cycles, for example over one or more past cycles. This factor can also be determined on the basis of statistical estimates of the duration of future cycles of the individual, for example on the basis of past measures and/or various predictive factors.

In order to obtain as many temperature values as there are "positionX" or "ZCM" values, the server carries out a normalization step on the temperature data recorded within the memory, by using a cubic spline polynomial interpolation so that every temperature readout gives 5 values in order to obtain a temperature value for each "positionX" and "ZCM" value versus time.

Subsequently, a step for suppressing the data generated by the portable module when it is not worn is carried out by the server. For this purpose, the server includes a program allowing during its execution, identification of the temperature values of less than 30°C as well as their time index, i.e. the time reference for the taking of the measurement. This algorithm suppresses from the memory the temperature values of less than 30°C as well as the "ZCM" and "positionX" values having the same time index (Fig. 2).

A step for correcting the values from the "positionX" tilt signal may be carried out in order to not be found with negative angular values when the portable module is worn upside down. For this, when the "positionX" value is greater than 90° than the program executed by the server carries out the following transformation by subtracting from 180 the "positionX" value: 180 - "positionX" value.

Subsequently, the server carries out an identification and zero reset step by the processor of the aberrant data from the "ZCM" and "positionX" signals. To do this, the processor independently carries out the following sub-steps on the whole of the "positionX" and "ZCM" data:

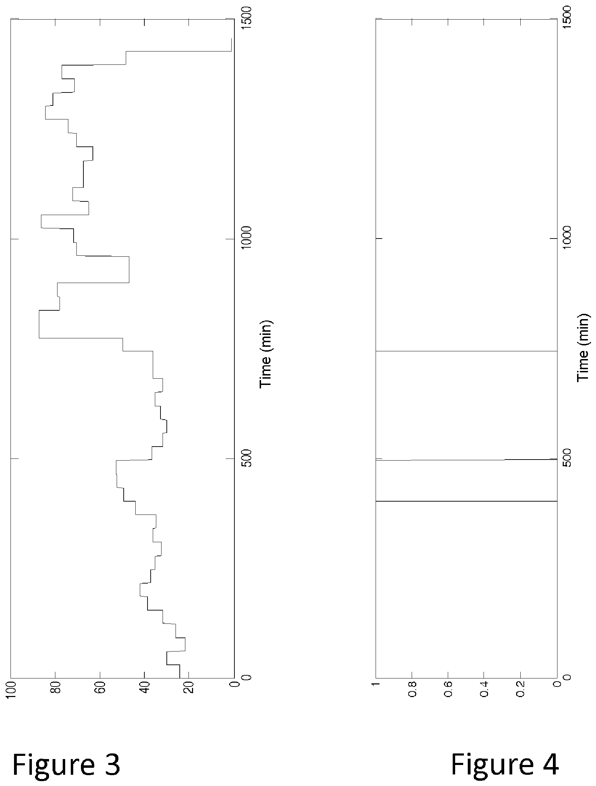

a) Selecting with the processor, in the memory of a first X block (for example, 31) consecutive values corresponding to a duration X (for example 31 minutes) of significant measurements,
b) Sorting in an increasing order the values of the block,
c) Suppressing value repetitions of the block, in order to obtain a unique data set,
d) Calculating the median of the first values of the unique data set,
e) Reading the block of X (31) values per Y sub-block (for example, 5) data corresponding to Y (respectively 5) measurement minutes, and resetting to zero in the memory of the Y sub-block (for example 5) data when there are less than Z <V (for example 2) values greater than the median,
f) Repeating steps a) to e) on the next block of consecutive values. (Fig. 3).

Following the step for removing the aberrant values "ZCM" and "positionX", the server transforms the values of the "positionX" and "ZCM" signals into binary values 0 or 1. The binary value 1 corresponds to a rest state and the binary value 0 to a state of activity.

In order to do this, on the whole of the positionX signal, the processor:

- determines and records in the memory, the minimum median by carrying out the following sub-steps:

  ◦ sorting the values in an increasing order,
  ◦ suppressing the value repetitions, in order to obtain a unique data set, and
  ◦ calculating the minimum median over the first X (31) values of the unique data set,

- determines and records in the memory, the maximum median by carrying out the following sub-steps:

  ◦ sorting the values in a decreasing order,
  ◦ suppressing the value repetitions, in order to obtain a unique data set, and
  ◦ calculating the maximum median over the first X (31) values of the unique data set,

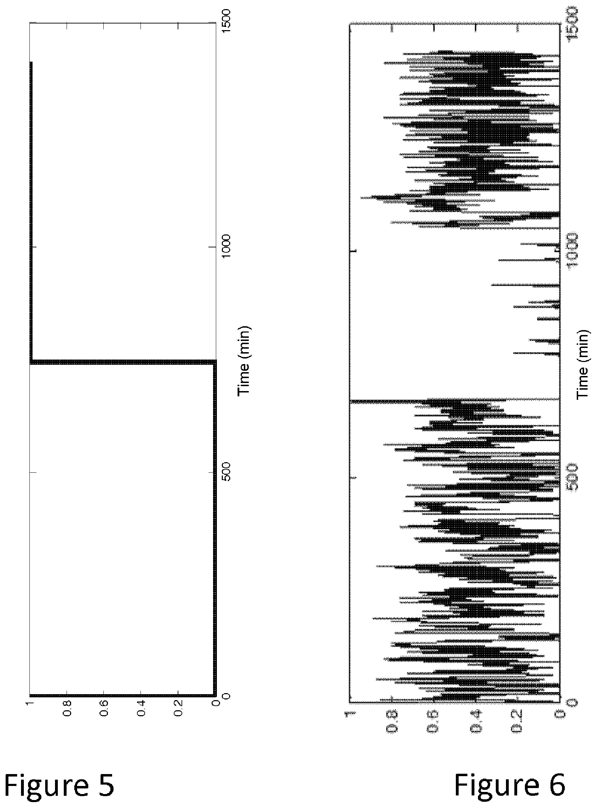

- determines and records in the memory, the threshold median corresponding to the average of the minimum and maximum medians,
- transforms and records, in the memory, the "positionX" data signal by replacing with 0 the values strictly less than the threshold median, and with 1 the values greater than or equal to the threshold median in order to obtain a positionX_binary data signal (Fig. 4),
- smooth out the values recorded in the memory of the positionX_binary data signal, when a set of data delimited by non-identical values corresponds to a time period of less than 1h30, the values of said data set are then reversed, from 0 to 1 and from1 to 0 (Fig. 5).

In other words, if the number of binary values contained in the set is less than 90, the binary values of said set are then reversed.

For the transformation into binary values of the "ZCM" values, the processor over the whole of the "ZCM" signal, carries out the following operations:

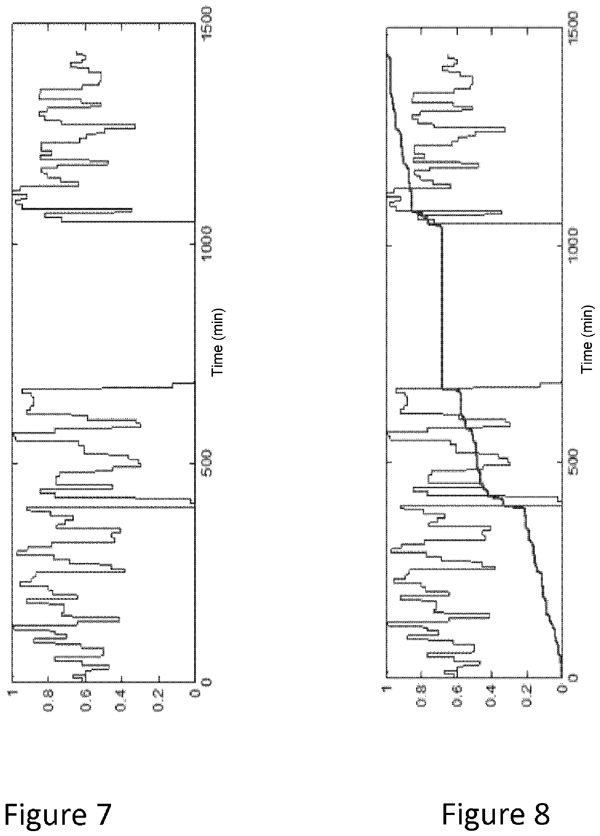

- normalization of the "ZCM" values by dividing each "ZCM" value by the maximum value of the signal in order to obtain a new set of data called ZCM_time for which the values vary between 0 and 1 (Fig. 6),
- application to the "ZCM_time" data set, of a variance filter over 10 points.
  Thus, in "ZCM-time" for each point for which the time index i will be modified, the variance filter will range from i to i+ 9 (Fig. 7).
  For example:

ZCM_time[0] =

Calculation_Variance(ZCM_time [0], ZCM_time[1],…,ZCM_time [9])

ZCM_time[1] =

Calculation_Variance(ZCM_time [1], ZCM_time[2],…,ZCM_time [10])

ZCM_time[2] =

Calculation_Variance(ZCM_time [2], ZCM_time[3],…,ZCM_time [11])

- a calculation of the accumulated quadratic average RMS from the ZCM_time signal, i.e. each RMS point (of time index i) is the sum of the preceding RMS point (with a time index i-1) with the result of the RMS over 2 ZCM_time points (Fig. 8).
For example:

RMS[0] = Calculation_RMS(ZCM_time[0], ZCM_time[1])

RMS[1] = Calculation_RMS(ZCM_time[1], ZCM_time[2]) + RMS[0]

RMS[2] = Calculation_RMS(ZCM_time[2], ZCM_time[3]) + RMS[1]

Also the processor, on the whole of the "ZCM" signal, carries out the following operations:

- seeking a plateau on the data of the accumulated quadratic average by carrying out the following sub-steps:

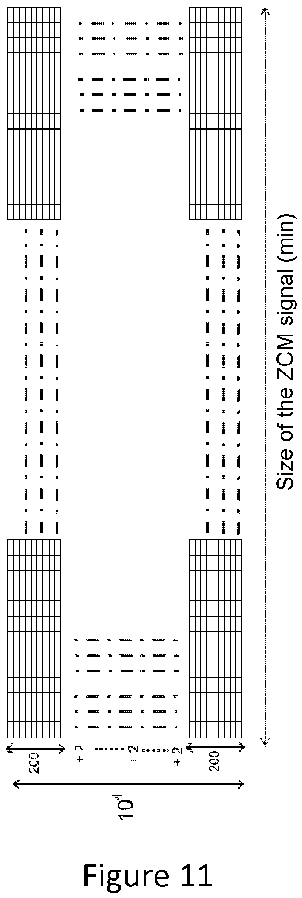

  ◦ multiplication of each value of the quadratic average by a factor of $10^4$ and calculation of the rounded to the nearest integer
  ◦ reading for example with a pitch of 2 units a corresponding window, for example with 200 units. In other words, the window gives the possibility of reading up to 200 values of the quadratic average at the same time and is displaced from two to two. I.e. the width of the window is 200 units, and its length is of the size of the signal. (Fig. 11)
  ◦ determination of the existence of a plateau when the window comprises at least 180 values of the quadratic average, the program executed by the processor then retains in memory the time index of the first and of the last point of the plateau.

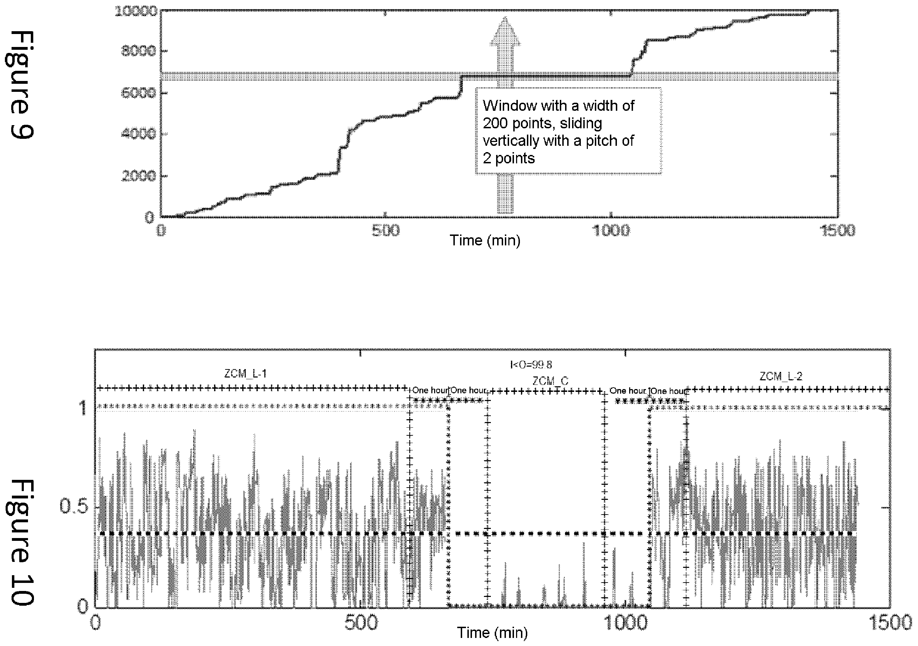

- transforms and records in the memory, the "ZCM" data signal by replacing the values located between the time indexes of the plateau with 1 and the other ones with 0 in order to obtain a "ZCM_binary" data signal (Fig. 9).

Following these binary transformation steps, the server compares the binary values of the "ZCM" and "positionX" signals and identifies at least one rest state of the individual, represented by a set of at least 180 binary values equals to 1.
Finally the server calculates the dichotomy index and proceeds with its display on the display means such as a screen, by applying the following equation:

$$I < O = (1 - \frac{NB_C}{NB_L}) \times 100$$

with:

- $NB_C$, the number of values of the first "ZCM" signal representative of the activity of the individual versus time, located in the lying-down state identified during step E, which are greater than the median of the values of the first "ZCM" signal representative of the activity of the individual versus time located outside the lying-down state identified during the preceding step,

- $NB_L$, the number of values of the first ZCM signal representative of the activity of the individual, located outside the lying-down state identified during the preceding step.

To do this, the server, by executing the program on its processor, carries out the following sub-steps:

- Identification of a set of "ZCM" values stored in memory under the name of "ZCM_C" and the points of which are contained in the plateau,
- Identification of a set of "ZCM" values stored in memory under the name of "ZCM_L", the points of which are on either side of the plateau,
- Calculation of the median, "Med_L", corresponding to the ZCM_L values.
- Counting in "ZCM_C", of the number of points, NB_C, above the median "Med_L" and in "ZCM_L", of the number of points, NB_L.
- Resolution of the equation of the dichotomy index.

In order to improve accuracy, in a preferred embodiment, the processor does not take into account for calculating the dichotomy index, the data corresponding to a time period corresponding to one hour before and one hour after the beginning of the plateau and one hour before and one hour after the end of the plateau. In other words, ZCM_C corresponds to the set of the ZCM values for which the points are contained in the plateau except for the first and last hour of the plateau, and ZCM_L corresponds to the set of ZCM values for which the points are on either side of the plateau except for one hour before the plateau and one hour just after the plateau.

Thus, in this particular embodiment, for calculating the dichotomy index, the latter is only calculated over a period of 20 hours per slice of 24 hours (Fig. 10).

According to another embodiment, if this is required in order to calculate the medians, a set of 16 values identical with the last value of the "ZCM" or "positionX" signal may be added at the end of the signals in order to obtain a sufficient number of values for carrying out the calculation of the medians.

Moreover, when the dichotomy index is greater than 97%, this means that the individual is well asleep and that his/her circadian rhythm is not perturbed.

This result represents a poor prognostic factor in terms of survival in hospitalized individuals, in particular for cancers notably when the dichotomy index is less than 97%.

Obtaining this result gives the possibility of knowing the circadian rhythm of an individual affected with cancer, which allows optimization of the time-modulated administration of the anticancer drugs thereby allowing improvement in tolerance and of the efficiency of the relevant anticancer drugs.

Such a method according to the invention gives the possibility of giving support to a physician with view to elaborating an optimum time-therapeutic scheme for each individual.

In addition, all the variables described above can all be dynamic variables (for example like the 24 hours period described above), which are defined on the basis of measured data and/or statistical estimates. Therefore, a a corrective factor can be added to or subtracted from them. Such corrective factor allows to take the individual variations between the patients into account for a given variable, for example as described herein.

The description of the present invention and the figures related thereto are not provided for limiting the scope of the invention but simply illustrate selected embodiments. One skilled in the art will understand that the technical features of a given embodiment according to a particularity, may in fact be combined with features of another embodiment according to another particularity unless the opposite is explicitly mentioned or if it is obvious that these features are incompatible. Further, the technical features described in a given embodiment according to a particularity, may be isolated from the other features of this embodiment unless the opposite is explicitly mentioned.

It should be obvious for those skilled in the art that the present invention allows embodiments under many other specific forms without departing from the field defined by the scope of the appended claims. They should be considered as an illustration and the invention should not be limited to the details given above.

**Claims**

1. A method for automatically determining the dichotomy index I<O of an individual, from at least data from a system comprising:

    - a portable module including :

        i. a temperature sensor generating a data signal representative of the body temperature of the individual,
        ii. an accelerometer simultaneously generating two data signals, a first "ZCM" signal, representative of the activity of the individual over time and a second "positionX" signal representative of the tilt of the individual with respect to the ascending vertical over time,

- computing resources including at least one processor and at least one memory, and configured for receiving the data signals from the portable sensor, and for carrying out said method,

**characterized in that** the method comprises at least the following steps performed by said processor :

A. receiving and recording in the memory of the various data of the temperature, ZCM and positionX signals of the portable module,
B. suppressing from the memory of the non-utilizable data from the module when the latter is not worn by the individual,
C. identifying and resetting to zero by the processor of the aberrant data from the ZCM and positionX signals of the accelerometer and of their recordings in the memory,
D. transforming the values of the positionX signal of the accelerometer into binary values (0 or 1) corresponding to an alert state (0) or lying-down state (1) of the individual and their recordings in the memory,
E. transforming the values of the ZCM signal of the accelerometer into binary values corresponding to an alert or lying-down state of the individual and their recordings in the memory,
F. comparing the binary values of the ZCM and positionX signals of the accelerometer and identifying at least one lying-down state of the individual constituted by an uninterrupted succession of a binary value identical for the signals of the accelerometer over a time period greater than or equal to 180 minutes,
G. calculating the dichotomy index and its display on a display means, by following the following equation:

$$I < O = (1 - \frac{NB_C}{NB_L}) \times 100$$

with:

- $NB_C$, the number of values of the first ZCM signal representative of the activity of the individual versus time, located in the lying-down state identified during step E, which are greater than the median of the values of the first ZCM signal representative of the activity of the individual versus time located outside the lying-down state identified during step E,
- $NB_L$, the number of values of the first signal relative to the activity (ZCM) of the individual, located outside the lying-down state identified during step E.

2. The method for automatically determining the dichotomy index I<O of an individual according to claim 1, **characterized in that** the computing resources are integrated in the portable module for carrying out said method.

3. The method for automatically determining the dichotomy index I<O of an individual according to claim 1, **characterized in that** the computing resources are integrated in a computer server distinct from the portable module.

4. The method for automatically determining the dichotomy index I<O of an individual according to claim 3, **characterized in that** the computer server comprises display means for displaying the results of the computation.

5. The method for automatically determining the dichotomy index I<O of an individual according to claim 1 or 2, **characterized in that** the data signals comprise values taken at a regular interval over a time period of 24 hours.

6. The method for automatically determining the dichotomy index I<O of an individual according one of claims 1 to 3, **characterized in that**, when the number of values of the data signals for the temperature sensor and the accelerometer are different, the processor carries out a normalization step on the data recorded in the memory prior to step B, by using a cubic spline polynomial interpolation so that each value from the temperature signal is assigned to a value of the positionX and ZCM signals, and said values are recorded in the memory of the system.

7. The method for automatically determining the dichotomy index I<O of an individual according to one of claims 1 to 4, **characterized in that**, during step B, the processor suppresses from the memory all the values of temperature data strictly less than 30°C and the values of the positionX and ZCM signals which are associated in time with the temperature value strictly less than 30°C.

8. The method for automatically determining the dichotomy index I<O of an individual according to claim 5, **characterized in that**, during step C, the processor independently applies on the whole of the positionX and ZCM data the following sub-steps:

   a) Selecting with the processor in the memory a first block of consecutive values,
   b) Sorting in an increasing order the values of the block,
   c) Suppressing value repetitions of the block, in order to obtain a unique data set,
   d) Calculating the median of the unique data set,
   e) Resetting to zero in the memory the values of the positionX and ZCM data signals when their value is greater than the median,
   f) Repeating steps a) to e) on the next block of consecutive values.

9. The method for automatically determining the dichotomy index I<O of an individual according to claim 6, **characterized in that**:

   • the blocks of values are constituted by 31 values corresponding to a time period of 31 minutes of the signals,
   • the median is calculated over the first 10 data of the unique data set,
   • the reset to zero is accomplished by sub-sets of 5 data corresponding to 5 consecutive minutes, if there are less than 2 values greater than the median, then the processor resets to zero in the memory the corresponding sub-set.

10. The method for automatically determining the dichotomy index I<O of an individual according to claim 7, **characterized in that**, during step D, on the whole of the positionX signal, the processor:

    • determines and records in the memory, the minimum median by carrying out the following sub-steps:

       ◦ sorting the values in an increasing order,
       ◦ suppressing the value repetitions, in order to obtain a unique data set, and
       ◦ calculating the minimum median over the first 31 values of the unique data set,

    • determines and records in the memory, the maximum median by carrying out the following sub-steps:

       ◦ sorting the values in a decreasing order,
       ◦ suppressing the value repetitions, in order to obtain a unique data set, and
       ◦ calculating the maximum median over the first 31 values of the unique data set,
       ◦ determines and records in the memory, the threshold median corresponding to the average of the minimum and maximum medians
       ◦ transforms and records, in the memory, the signal of positionX data by replacing with 0 the values strictly less than the threshold median, and with 1 the values greater than or equal to the latter in order to obtain a positionX_binary data signal,
       ◦ smoothes out the values recorded in the memory of the positionX_binary data signal, by the processor when a set of data delimited by non-identical values corresponds to a time period of less than 1h30, the values of said data set are then reversed, from 0 to 1 or from 1 to 0.

11. The method for automatically determining the dichotomy index I<O of an individual according to claim 8, **characterized in that**, during step E, on the whole of the ZCM signal, the processor

    • normalizes the ZCM values by dividing each ZCM value by the maximum value of the signal in order to obtain a new ZCM_time signal, the values of which vary between 0 and 1,
    • applies to the ZCM_time signal a variance filter over 10 points and records the signal in the memory,
    • calculates the accumulated quadratic average from the ZCM_time signal,
    • searches for a plateau on the data of the accumulated quadratic average by carrying out with the processor the following sub-steps:

       ◦ multiplication of each value of the quadratic average by a factor of $10^4$ and calculating the rounded to the nearest integer,
       ◦ reading with a pitch of 2 units a window corresponding to 200 units,
       ◦ determining the number of points in each window, if the number of found points corresponds to a time period greater than 180 min, the processor increments a "plateau" counter and retains in memory the time index of the first and of the last point of the plateau,

◦ transforming and recording in the memory, the ZCM data signal by replacing the values located between the time indexes of the plateau with 1 and the other ones with 0 in order to obtain a ZCM_binary data signal.

12. The method for automatically determining the dichotomy index I<O of an individual according to claim 9, **characterized in that**, during step F, the processor carries out a comparison of the ZCM_binary and positionX_binary data signals in order to determine the time indexes of the plateau for which the initial and final data values correspond to 1, the lying-down state.

13. The method for automatically determining the dichotomy index I<O of an individual according to claim 10, **characterized in that**, during step G, the processor does not take into account for calculating the dichotomy index, the data corresponding to a time period of one hour before and of one hour after the beginning of the plateau and one hour before and one hour after the end of the plateau.


**Patentansprüche**

1. Verfahren zur automatischen Bestimmung des Dichotomie-Index I<O eines Individuums, aus mindestens Daten von einem System, das Folgendes umfasst:

 - ein tragbares Modul umfassend:

  i. einen Temperatursensor, der ein Datensignal erzeugt, das für die Körpertemperatur des Individuums repräsentativ ist,
  ii. einen Beschleunigungssensor, der gleichzeitig zwei Datensignale erzeugt, ein erstes "ZCM"-Signal, das für die Aktivität des Individuums im Laufe der Zeit repräsentativ ist und ein zweites "positionX"-Signal, das für die Neigung des Individuums in Bezug auf die aufsteigende Vertikale im Laufe der Zeit repräsentativ ist,

 - Computerressourcen umfassend mindestens einen Prozessor und mindestens einen Speicher, die zum Empfangen der Datensignalen von dem tragbaren Sensor und zum Ausführen des genannten Verfahrens eingestellt sind,

 **dadurch gekennzeichnet, dass** das Verfahren mindestens die folgenden Schritte umfasst, die von dem genannten Prozessor ausgeführt werden:

  A. das Empfangen und die Aufnahme in den Speicher der verschiedenen Daten der Temperatur-, ZCM- und positionX-Signale des tragbaren Moduls,
  B. das Löschen aus dem Speicher der nicht verwendbaren Daten des Modul, wenn dieses von dem Individuums nicht getragen wird,
  C. das Erkennen und Zurücksetzen auf Null durch den Prozessor der aberranten Daten von den ZCM- und positionX-Signalen des Beschleunigungssensors und deren Aufnahmen in den Speicher,
  D. das Umwandeln der Werte des positionX-Signals des Beschleunigungssensors in Binärwerte (0 oder 1), die dem Wachzustand (0) oder dem Ruhezustand (1) des Individuums entsprechen und deren Aufnahmen in den Speicher,
  E. das Umwandeln der Werte des ZCM-Signals des Beschleunigungssensors in Binärwerte, die dem Wachzustand oder dem Ruhezustand des Individuums entsprechen und deren Aufnahmen in den Speicher,
  F. das Vergleichen der Binärwerte der ZCM- und positionX-Signale des Beschleunigungssensors und das Erkennen mindestens eines Ruhezustands bestehend aus einer ununterbrochenen Aufeinanderfolge eines identischen Binärwerts für die Signale des Beschleunigungssensors über einen Zeitraum von mehr als oder gleich 180 Minuten,
  G. das Berechnen des Dichotomie-Index und seine Anzeige auf einem Anzeigemittel gemäß der folgenden Gleichung:

$$I < 0 = \left(1 - \frac{NB_C}{NB_L}\right) \times 100$$

mit:

- $NB_C$, die Anzahl der Werte des ersten ZCM-Signals, das für die Aktivität des Individuums über der Zeit repräsentativ ist, innerhalb des während Schritt E identifizierten Ruhezustands, die größer als der Medianwert der Werte des ersten ZCM-Signals ist, das für die Aktivität des Individuums über der Zeit repräsentativ ist, außerhalb des während Schritt E identifizierten Ruhezustands,
- $NB_L$, die Anzahl der Werte des ersten Signals außerhalb des während Schritt E identifizierten Ruhezustands in Bezug auf die Aktivität (ZCM) des Individuums.

2. Verfahren zur automatischen Bestimmung des Dichotomie-Index I<O eines Individuums nach Anspruch 1, **dadurch gekennzeichnet, dass** die Computerressourcen in dem tragbaren Modul zum Ausführen des Verfahrens integriert sind.

3. Verfahren zur automatischen Bestimmung des Dichotomie-Index I<O eines Individuums nach Anspruch 1, **dadurch gekennzeichnet, dass** die Computerressourcen in einem von dem tragbaren Modul unterschiedlichen Computerserver integriert sind.

4. Verfahren zur automatischen Bestimmung des Dichotomie-Index I<O eines Individuums nach Anspruch 3, **dadurch gekennzeichnet, dass** der Computerserver Anzeigemittel zum Anzeigen der Ergebnisse der Berechnung umfasst.

5. Verfahren zur automatischen Bestimmung des Dichotomie-Index I<O eines Individuums nach Anspruch 1 oder 2, **dadurch gekennzeichnet, dass** die Datensignale Werte umfassen, die in regelmäßigen Abständen über einen Zeitraum von 24 Stunden aufgenommen werden.

6. Verfahren zur automatischen Bestimmung des Dichotomie-Index I<O eines Individuums nach einem der Ansprüche 1 bis 3, **dadurch gekennzeichnet, dass** der Prozessor vor Schritt B einen Normalisierungsschritt auf die in dem Speicher aufgenommenen Daten ausführt, wenn die Anzahl von Werten der Datensignale für den Temperatursensor und den Beschleunigungssensor unterschiedlich sind, durch Verwendung einer kubischen Spline-Polynom-Interpolation, sodass jeder Wert des Temperatursignals einem Wert der positionX- und ZCM-Signale zugeordnet wird, und die genannten Werte in den Speicher aufgenommen werden.

7. Verfahren zur automatischen Bestimmung des Dichotomie-Index I<O eines Individuums nach einem der Ansprüche 1 bis 4, **dadurch gekennzeichnet, dass** während Schritt B, der Prozessor alle Werte der Temperaturdaten, die grundsätzlich unter 30°C sind und die Werte des positionX- und ZCM-Signale, die zeitlich mit dem Temperaturwert grundsätzlich unter 30°C verbunden sind, aus dem Speicher löscht.

8. Verfahren zur automatischen Bestimmung des Dichotomie-Index I<O eines Individuums nach Anspruch 5, **dadurch gekennzeichnet, dass**, während Schritt C, der Prozessor selbstständig die folgenden Unterschritte auf die gesamten positionX- und ZCM-Daten anwendet:

a) das Auswählen eines ersten Blocks aufeinanderfolgender Werte mit dem Prozessor in dem Speicher,
b) das Sortieren in aufsteigender Reihenfolge der Werte des Blocks,
c) das Löschen der Wiederholungen des Blocks, um einen einzelnen Datensatz zu erhalten,
d) das Berechnen des Medianwerts des einzelnen Datensatzes,
e) das Zurücksetzen auf Null der Werte der positionX- und ZCM-Datensignale in dem Speicher, wenn ihr Wert größer als der Medianwert ist,
f) das Wiederholen der Schritte a) bis e) auf den nächsten Block aufeinanderfolgender Werte.

9. Verfahren zur automatischen Bestimmung des Dichotomie-Index I<O eines Individuums nach Anspruch 6, **dadurch gekennzeichnet, dass**:

• die Wertblöcke aus 31 Werte bestehen, die einer Zeitspanne von 31 Minuten des Signals entsprechen,
• der Medianwert über die ersten 10 Daten des einzelnen Datensatzes berechnet wird,
• das Zurücksetzen auf Null von Untersätzen von 5 Daten ausgeführt wird, die 5 aufeinanderfolgenden Minuten entsprechen, wenn es weniger als 2 Werte gibt, die größer als der Medianwert sind, dann setzt der Prozessor den entsprechenden Untersatz in dem Speicher auf Null zurück.

10. Verfahren zur automatischen Bestimmung des Dichotomie-Index I<O eines Individuums nach Anspruch 7, **dadurch**

**gekennzeichnet, dass**, während Schritt D, der Prozessor auf das gesamte positionX-Signal:

• durch das Anwenden der folgenden Unterschritte den minimalen Medianwert bestimmt und in den Speicher aufnimmt:

◦ das Sortieren in aufsteigender Reihenfolge der Werte,
◦ das Löschen der Wiederholungen der Werte, um einen einzelnen Datensatz zu erhalten, und
◦ das Berechnen des minimalen Medianwertes über die ersten 31 Werte des einzelnen Datensatzes,

• durch das Anwenden der folgenden Unterschritte den maximalen Medianwert bestimmt und in den Speicher aufnimmt:

◦ das Sortieren in absteigender Reihenfolge der Werte,
◦ das Löschen der Wiederholungen der Werte, um einen einzelnen Datensatz zu erhalten, und
◦ das Berechnen des maximalen Medianwertes über die ersten 31 Werte des einzelnen Datensatzes,
◦ den Schwellenmedianwert bestimmen und in den Speicher aufnehmen, der dem Mittelwert des minimalen und maximalen Medianwerte entspricht
◦ das Signal der positionX-Daten umwandelt und in den Speicher aufnimmt, durch das Ersetzen mit 0 der Werte, die grundsätzlich kleiner als der Schwellenmedianwert sind, und mit 1, die Werte, die grundsätzlich größer als der Schwellenmedianwert oder gleich sind, um ein positionX_binary-Datensignal zu erhalten,
◦ die in dem Speicher aufgenommenen Werte des positionX_binary-Datensignals durch den Prozessor glättet, wenn ein Datensatz, der durch nicht identische Werte begrenzt ist, einen Zeitraum von weniger als 1h30 entspricht, dann werden die Werte des Datensatzes umgekehrt, von 0 auf 1 oder von 1 auf 0 gesetzt.

11. Verfahren zur automatischen Bestimmung des Dichotomie-Index I<O eines Individuums nach Anspruch 8, **dadurch gekennzeichnet, dass**, während Schritt E, der Prozessor auf das gesamte ZCM-Signal:

• die ZCM-Werte normalisiert, indem er jeden ZCM-Wert durch den maximalen Wert des Signals dividiert, um ein neues ZCM_time-Signal zu erhalten, wobei deren Werte zwischen 0 und 1 variieren,
• auf das ZCM_time-Signal einen Varianzfilter über 10 Punkte anwendet und das Signal in den Speicher aufnimmt,
• den akkumulierten quadratischen Mittelwert des ZCM_time-Signal berechnet,
• anhand der Daten des akkumulierten quadratischen Mittelwerts durch das Anwenden mit dem Prozessor der folgenden Unterschritte nach einem Plateau sucht:

◦ Multiplikation von jedem Wert des quadratischen Mittelwertes mit dem Faktor $10^4$ und Berechnung der auf die nächste ganze Zahl gerundeten Zahl,
◦ Lesen eines Zeitfensters mit einer Teilung von 2 Einheiten, das 200 Einheiten entspricht
◦ das Bestimmen der Anzahl von Punkten in jedem Zeitfenster, wenn die Anzahl der gefundenen Punkte einem Zeitraum von mehr als 180 Minuten entspricht, inkrementiert der Prozessor einen "Plateau"-Zähler und speichert den Zeitindex des erstens und des letzten Punktes des Plateaus,
◦ das Umwandeln und die Aufnahme in den Speicher des ZCM-Datensignals durch das Ersetzen der Werte innerhalb der Zeitindex des Plateaus durch 1 und die andere durch 0 um ein ZCM_binary-Datensignal zu erhalten.

12. Verfahren zur automatischen Bestimmung des Dichotomie-Index I<O eines Individuums nach Anspruch 9, **dadurch gekennzeichnet, dass**, während Schritt F, der Prozessor einen Vergleich der ZCM_binary- und positionX_binary-Datensignale durchführt, um die Zeitindizes des Plateaus zu bestimmen, für die die Anfangs- und Enddatenwerte 1, des Ruhezustands, entsprechen.

13. Verfahren zur automatischen Bestimmung des Dichotomie-Index I<O eines Individuums nach Anspruch 10, **dadurch gekennzeichnet, dass**, während Schritt G, der Prozessor um das Dichotomie-Index zu berechnen die Daten, die einer Zeitspanne von einer Stunde vor und nach dem Anfang des Plateaus und einer Stunde vor und nach dem Ende des Plateaus entsprechen, nicht berücksichtigt.

**Revendications**

1.  Procédé pour déterminer automatiquement l'indice de dichotomie I < O d'un individu, à partir d'au moins des données d'un système comprenant :

    - un module portable comportant :

        i. un détecteur de température générant un signal de données représentant la température corporelle de l'individu,
        ii. un accéléromètre générant simultanément deux signaux de données, un premier signal « ZCM », représentant l'activité de l'individu dans le temps et un deuxième signal de « de position X » représentant le basculement de l'individu par rapport à une ascendante verticale dans le temps,

        - des ressources informatiques comportant au moins un processeur et au moins une mémoire, et configurées pour recevoir les signaux de données à partir du détecteur portable, et pour mettre en oeuvre ledit procédé,

    **caractérisé en ce que** le procédé comprend au moins les étapes suivantes effectuées par ledit processeur :

        A. recevoir et enregistrer dans la mémoire diverses données des signaux de température, ZCM et de position X du module portable,
        B. supprimer de la mémoire les données inutilisables du module lorsque ce dernier n'est pas porté par l'individu,
        C. identifier et remettre à zéro par le processeur les données aberrantes des signaux ZCM et de position X de l'accéléromètre et de leurs enregistrements dans la mémoire,
        D. transformer les valeurs du signal de position X de l'accéléromètre en valeurs binaires (0 ou 1) correspondant à un état d'alerte (0) à un état d'allongement (1) de l'individu et à leurs enregistrements dans la mémoire,
        E. transformer les valeurs du signal ZCM de l'accéléromètre en valeurs binaires correspondant à des états d'alerte ou d'allongement de l'individu et leurs enregistrements dans la mémoire,
        F. comparer les valeurs binaires des signaux ZCM et de position X de l'accéléromètre et identifier au moins un état d'allongement de l'individu constitué d'une succession ininterrompue d'une valeur binaire identique pour les signaux de l'accéléromètre sur une période de temps supérieure ou égale à 180 minutes,
        G. calculer l'indice de dichotomie et son affichage sur un moyen d'affichage, en suivant l'équation suivante :

        $$I < 0 = \left(1 - \frac{NB_c}{NB_L}\right) \times 100$$

        avec :

            - NBc, le nombre de valeurs du premier signal ZCM représentant l'activité de l'individu en fonction du temps, situées dans l'état d'allongement identifié au cours de l'étape E, qui sont supérieures à la médiane des valeurs du premier signal ZCM représentant l'activité de l'individu en fonction du temps situées en dehors de l'état d'allongement identifié au cours de l'étape E,
            - $NB_L$, le nombre de valeurs du premier signal relatif à l'activité (ZCM) de l'individu, situées en dehors de l'état d'allongement identifié au cours de l'étape E.

2.  Procédé pour déterminer automatiquement l'indice de dichotomie I < O d'un individu selon la revendication 1, **caractérisé en ce que** les ressources informatiques sont intégrées dans le module portable pour mettre en oeuvre ledit procédé.

3.  Procédé pour déterminer automatiquement l'indice de dichotomie I < O d'un individu selon la revendication 1, **caractérisé en ce que** les ressources informatiques sont intégrées dans un serveur informatique distinct du module portable.

4.  Procédé pour déterminer automatiquement l'indice de dichotomie I < O d'un individu selon la revendication 3, **caractérisé en ce que** le serveur informatique comprend un moyen d'affichage pour afficher les résultats du calcul.

5.  Procédé pour déterminer automatiquement l'indice de dichotomie I < O d'un individu selon la revendication 1 ou 2, **caractérisé en ce que** les signaux de données comprennent des valeurs prises à intervalles réguliers sur une période de temps de 24 heures.

6. Procédé pour déterminer automatiquement l'indice de dichotomie I < O d'un individu selon l'une des revendications 1 à 3, **caractérisé en ce que**, lorsque les nombres de valeurs des signaux de données pour le détecteur de température et l'accéléromètre sont différents, le processeur met en oeuvre une étape de normalisation des données enregistrées dans la mémoire avant l'étape B, en utilisant une interpolation polynomiale de fonction spline cubique de sorte que chaque valeur du signal de température soit attribuée à une valeur des signaux de position X et ZCM, et que lesdites valeurs soient enregistrées dans la mémoire du système.

7. Procédé pour déterminer automatiquement l'indice de dichotomie I < O d'un individu selon l'une des revendications 1 à 4, **caractérisé en ce que**, au cours de l'étape B, le processeur supprime de la mémoire toutes les valeurs de données de température strictement inférieures à 30°C et les valeurs des signaux de position X et ZCM qui sont associées dans le temps à la valeur de température strictement inférieure à 30°C.

8. Procédé pour déterminer automatiquement l'indice de dichotomie I < O d'un individu selon la revendication 5, **caractérisé en ce que**, au cours de l'étape C, le processeur applique indépendamment sur la totalité des données de position X et ZCM les sous-étapes suivantes :

   a) sélectionner à l'aide du processeur dans la mémoire un premier bloc de valeurs consécutives,
   b) trier dans un ordre croissant les valeurs du bloc,
   c) supprimer des répétitions de valeurs du bloc, afin d'obtenir un ensemble de données unique,
   d) calculer la médiane de l'ensemble de données unique,
   e) remettre à zéro dans la mémoire les valeurs des signaux de données de position X et ZCM lorsque leur valeur est supérieure à la médiane,
   f) répéter les étapes a) à e) sur le bloc suivant de valeurs consécutives.

9. Procédé pour déterminer automatiquement l'indice de dichotomie I < O d'un individu selon la revendication 6, **caractérisé en ce que** :

   • les blocs de valeurs sont constitués de 31 valeurs correspondant à une période de temps des signaux de 31 minutes,
   • la médiane est calculée sur les premières 10 données de l'ensemble de données unique,
   • la remise à zéro est accomplie par des sous-ensembles de 5 données correspondant à 5 minutes consécutives, s'il y a moins de 2 valeurs supérieures à la médiane, le processeur remet alors à zéro dans la mémoire les sous-ensembles correspondants.

10. Procédé pour déterminer automatiquement l'indice de dichotomie I < O d'un individu selon la revendication 7, **caractérisé en ce que**, au cours de l'étape D, sur la totalité du signal de position X, le processeur :

   • détermine et enregistre dans la mémoire, la médiane minimale en mettant en oeuvre les sous-étapes suivantes :

     ◦ trier les valeurs dans un ordre croissant,
     ◦ supprimer les répétitions de valeurs, afin d'obtenir un ensemble de données unique, et
     ◦ calculer la médiane minimale sur les premières 31 valeurs de l'ensemble de données unique,

   • détermine et enregistre dans la mémoire, la médiane maximale en mettant en oeuvre les sous-étapes suivantes :

     ◦ trier les valeurs dans un ordre décroissant,
     ◦ supprimer les répétitions de valeurs, afin d'obtenir un ensemble de données unique, et
     ◦ calculer la médiane maximale sur les premières 31 valeurs de l'ensemble de données unique,
     ◦ déterminer et enregistrer dans la mémoire, la médiane seuil correspondant à la moyenne des médianes minimale et maximale,
     ◦ transformer et enregistrer, dans la mémoire, le signal de données de position X en remplaçant par 0 les valeurs strictement inférieures à la médiane seuil, et par 1 les valeurs supérieures ou égales à cette dernière afin d'obtenir un signal de données binaires de position X,
     ◦ aplanir les valeurs enregistrées dans la mémoire du signal de données binaires de position X, par le processeur lorsqu'un ensemble de données délimitées par des valeurs non identiques correspond à une période de temps inférieure à 1h30, les valeurs dudit ensemble de données sont alors inversées, de 0 à 1 ou de 1 à 0.

**11.** Procédé pour déterminer automatiquement l'indice de dichotomie I < O d'un individu selon la revendication 8, **caractérisé en ce que**, au cours de l'étape E, sur la totalité du signal ZCM, le processeur

- normalise les valeurs ZCM en divisant chaque valeur ZCM par la valeur maximale du signal afin d'obtenir un nouveau signal temporel ZCM, dont les valeurs varient entre 0 et 1,
- applique au signal temporel ZCM un filtre des écarts sur 10 points et enregistre le signal dans la mémoire,
- calcule la moyenne quadratique cumulée à partir du signal temporel ZCM,
- cherche un plateau sur les données de la moyenne quadratique cumulée en mettant en oeuvre à l'aide du processeur les sous-étapes suivantes :

  ◦ multiplier chaque valeur de la moyenne quadratique par un facteur de $10^4$ et calculer l'arrondi à l'entier le plus proche,
  ◦ lire avec un pas de 2 unités une fenêtre correspondant à 200 unités,
  ◦ déterminer le nombre de points dans chaque fenêtre, si le nombre de points trouvé correspond à une période de temps supérieure à 180 min, le processeur incrémente un compteur de "plateau" et retient dans mémoire l'indice temporel du premier et du dernier point du plateau,
  ◦ transformer et enregistrer dans la mémoire, le signal de données ZCM en remplaçant les valeurs situées entre les indices temporels du plateau par 1 et les autres par 0 afin d'obtenir un signal de données binaires ZCM.

**12.** Procédé pour déterminer automatiquement l'indice de dichotomie I < O d'un individu selon la revendication 9, **caractérisé en ce que**, au cours de l'étape F, le processeur met en oeuvre une comparaison des signaux de données binaires ZCM et binaires de position X afin de déterminer les indices temporels du plateau pour lequel les valeurs de données initiales et finales correspondent à 1, l'état d'allongement.

**13.** Procédé pour déterminer automatiquement l'indice de dichotomie I < O d'un individu selon la revendication 10, **caractérisé en ce que**, au cours de l'étape G, le processeur ne prend pas en compte pour calculer l'indice de dichotomie, les données correspondant à une période de temps d'une heure avant et d'une heure après le début de plateau et d'une heure avant et d'une heure après la fin du plateau.

Figure 1

Figure 2

Figure 3

Figure 4

Figure 5

Figure 6

Figure 7

Figure 8

Figure 9

Figure 10

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014210588 A1
- WO 2015082382 A1
- US 20080117060 A1
- US 20140215246 A1

### Non-patent literature cited in the description

- **TILMANNE, J.** algorithms for sleep-wake identification using actigraphy: a comparative study and new results. *Journal of Sleep Research,* 2009, vol. 18 (1), 85-98